# EUROPEAN PATENT SPECIFICATION

(11) **EP 4 120 893 B1**
(45) Date of publication and mention of the grant of the patent: **11.02.2026**
(21) Application number: 21713650.6
(22) Date of filing: 18.03.2021
(51) Int. Cl.: A61B 5/00, A61M 60/178, A61M 60/196, A61M 60/216, A61M 60/531, A61M 60/816, A61M 60/857

(54) **PRESSURE SENSOR ARRANGEMENT AND METHOD**
DRUCKSENSORANORDNUNG UND -VERFAHREN
AGENCEMENT DE CAPTEUR DE PRESSION ET PROCÉDÉ

(30) Priority: 18.03.2020 SE 2050298
(43) Date of publication of application: 25.01.2023
(73) Proprietor: Scandinavian Real Heart AB, 722 13 Västerås (SE)
(72) Inventor: NAJAR, Azad, 723 41 Västerås (SE); BRYNEDAL IGNELL, Nils, 722 24 Västerås (SE)
(74) Representative: Valea AB
(86) International application number: PCT/EP2021/057034
(87) International publication number: WO 2021/186009

(56) References cited:
- EP-A1- 3 222 206
- WO-A1-01/48451
- US-A1- 2003 045 772
- US-A1- 2011 066 046
- US-B2- 9 713 662

## Description

### TECHNICAL FIELD

Generally, the present invention relates to a pressure senor arrangement suitable for heart prosthesis (total artificial heart), heart assist pumps (ventricular assist devices: VADs, LVAD, etc.) or as an implant for measuring venous and arterial pressure in either the pulmonary or systemic circulations.

### BACKGROUND

Document US 2011/066046 A1 discloses an implantable flat blood pressure sensing cuff structure and an implantable blood pressure monitoring device.

A blood-pumping device or artificial heart is disclosed for example in WO 2016/020219 or WO 2017/137486, by the same applicant.

Other blood pumps are known, for example through US 2003045772 and US 20190351118.

Despite steady progress in developing a permanent artificial heart for long-term implantation in a patient as a substitute for a failed natural heart, a number of issues must still be resolved. Among the issues that need to be addressed in an untethered artificial heart system are control strategies that respond to varying physiological demands, and mechanisms for accommodating the flow imbalance between the pulmonary and systemic circulations.

Left-right cardiac output differences have been well documented. Physiologically, the blood flow pumped by the left side of the heart is higher than that pumped by the right side of the heart. This difference is largely attributable to a circulatory pathway known as the bronchial shunt. This flow originates in the left arterial system, passes through the bronchial tissue, and then returns directly to the left atrium. This difference typically appears to be up to about ten percent of cardiac output with the left-side flow always greater than the right-side flow. Artificial heart systems must account for this inherent physiological circulatory imbalance. In addition, sources of flow imbalance can be fabricated. For example, differences in regurgitation through artificial valves provided on the left and right sides can introduce a flow imbalance. Artificial heart systems must account for these types of circulating imbalances as well.

### SUMMARY

There is a need for a pressure sensor that allows measuring pressure and specially blood pressure, which can be arranged on or in communication with heart prothesis, Total Artificial Heart (TAH), heart assist pumps, such as ventricular assist devices: VADs, LVAD, etc. or as an implant for measuring venous and arterial pressure in both pulmonary and systemic circulations. The invention also relates to a controller, which based on the pressure values obtained from the pressure sensor can automatically control parameters such as the stroke rate and stroke volume of the blood pump.

For these reasons a pressure sensor is provided, comprising a biocompatible housing, a biocompatible flexible membrane covering an open portion in the housing, a pressure transferring medium, an attachment ring, an electrical connection, and a pressure sensitive sensor. The sensor housing maybe made of hard polyurethane, hard silicone, biocompatible metal such as titanium or stainless steel or any other biocompatible stiff material. The attachment ring maybe made of a biocompatible material, such as Dacron, ePTFE, polyester or other material that can be healed together with the natural tissues. The pressure sensitive sensor may comprise MEMS (small-scale microelectromechanical system) sensor or nanoelectromechanical system (NEMS). The flexible membrane may be made of polyurethane, titanium, silicone or any other blood compatible material. The pressure transferring medium in the housing may consist of a biocompatible, implantable oil, such as medically graded silicone oil. The sensor is intended for use in or in communication with one or several of heart prostheses, total artificial heart, heart assist pumps, as an implant for measuring venous and arterial pressure in pulmonary and systemic circulations, thorax pressure measurement, kidneys, urinary bladder, or pressure measured in the abdominal cavity.

The invention also relates to a controller unit for controlling a cardiac prosthesis, the prosthesis comprising: at least one pump portion; an inlet connected to said at least one pump portion; an outlet connected to said at least one pump portion; a pressure sensor as described above and configured to measure pressure of a fluid flowing from the inlet to the outlet; a pump actuator configured to induce the flow of the fluid flow, and the controller unit further comprising a memory and a processing unit. The controller unit is configured to: obtain a pressure value from the pressure sensor, obtain a desired value for the pressure of the fluid flowing into the pump, calculate an error signal equal to the difference of the desired value for the pressure and the measured pressure, and control the output of the pump such that the measured pressure is near or equal to the desired pressure, by controlling a pump stroke rate and/or a pump stroke volume.

The invention also relates to a cardiac prosthesis comprising a pressure sensor as described.

### BRIEF DESCRIPTION OF THE DRAWINGS

Reference is made to the attached drawings, wherein elements having the same reference number designation may represent like elements throughout.
Fig. 1 is a schematic perspective view of a pressure sensor according to one exemplary embodiment;
Fig. 2 is a schematic cross-sectional view of the pressure sensor of Fig. 1;
Fig. 3 is a first exemplary application embodiment of a pressure sensor according to the present invention;
Fig. 4 is a second exemplary application embodiment of a pressure sensor according to the present invention;
Figs. 5 and 6 illustrate a third exemplary application embodiment of a pressure sensor according to the present invention;
Figs. 7a and 7b illustrate a fourth exemplary application embodiment of a pressure sensor according to the present invention;
Fig. 8 is a schematic diagram of a controller in accordance with one aspect of the invention;
Fig. 9 is a schematic diagram of a control unit according to the present invention.

### DETAILED DESCRIPTION

The following detailed description refers to the accompanying drawings.

The term artificial heart as used herein may relate to a pumping device connectable to a subject.

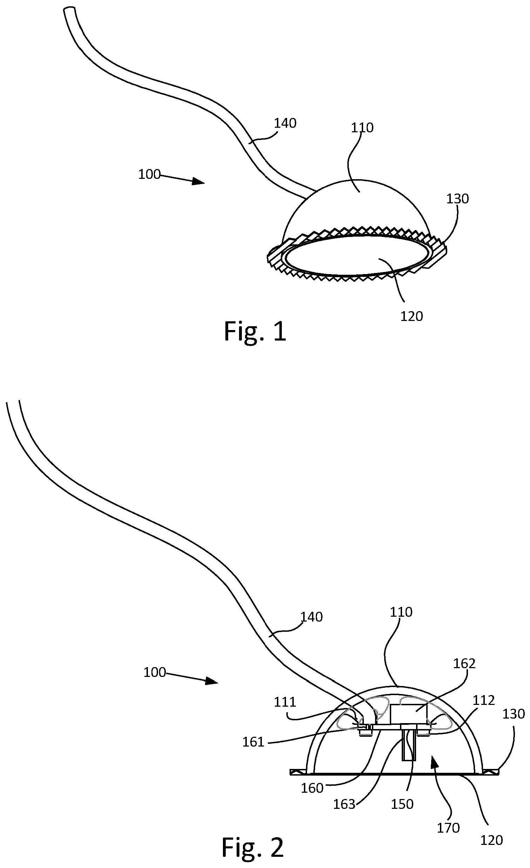

Fig. 1 illustrates a pressure sensor 100, according to one exemplary embodiment of the present invention, from below. The pressure sensor comprises a substantially domeshaped housing 110, a pressure sensitive elastic membrane 120, an attachment ring 130 and an electrical connection 140.

The pressure sensor 100 is detailed in the cross-sectional view of Fig. 2.

A pressure sensitive sensor 150 is arranged on a circuit board 160, also carrying electronics 162 and connections to the electrical connection 140.

The inner surface of the housing 110 comprises abutments 111 comprising screw holes for receiving screws 112. The circuit board is attached to the inner surface of the housing 110 by means of screws 112. Clearly other attachment means, such as adhesives may also be used for attaching the circuit board to the housing.

A direction pipe 163 may be arranged on one surface of the circuit board. The lumen of the direction pipe is in communication with the surface of the pressure sensitive sensor 150. However, in some exemplary embodiments, the direction pipe can be excluded.

The space 170 in the housing is entirely filled with a pressure transferring medium.

The sensor housing 110 may be made of hard polyurethane, hard silicone, biocompatible metal such as titanium or stainless steel or any other biocompatible stiff material.

The attachment ring 130, which entirely or partly surrounds the lower portion of the housing may be made of Dacron, ePTFE, polyester or other material that can be healed together with the natural tissues.

The pressure sensitive sensor 150 may comprise of MEMS (small-scale microelectromechanical system) sensor, nanoelectromechanical systems (NEMS), or any other suitable pressure sensor architecture.

The flexible thin membrane 120 is sealed in a leakage-free way, e.g., using an adhesive agent or a sealing washer to the housing 110 and may be made of polyurethane, silicone, titanium or any other blood compatible material. Consequently, the sensor body is hermetically sealed.

The pressure transferring medium, or agent filled in the space 170 of the housing 110 may consist of a biocompatible, implantable oil, such as medical graded silicone oil, or any other biocompatible, medical graded fluid.

In operation, when the membrane 120 is affected by a pressure, e.g., of the blood, the flexible membrane 120 will either bulge when the pressure on the membrane increases or it will bow outwards when the pressure decreases. In this way, the pressure transfer medium inside the housing is compressed or expanded due to the influence of the pressure on the flexible membrane 120. The pressure is transferred by the pressure transferring medium inside the pipe 163 (if present) from the membrane to the sensor's 150 surface. The sensitive surface of the MEMS sensor is thus affected by the pressure changing in the pressure transfer medium and the MEMS sensor translates the pressure to a digital value and an electrical signal representing the pressure is generated and can be provided to a controller.

The described and illustrated pressure sensor is given as an example. The sensor housing and form may vary in various ways depending on the application area requirements. The sensor portion, electronics and connection may also vary depending on the application area. A wireless transmission ability may also be included.

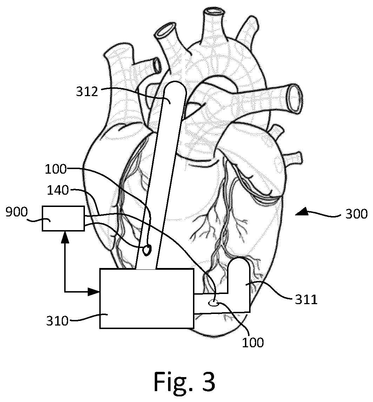

Fig. 3 illustrates a first example of an application of the pressure sensor described previously. In this case, a heart 300 of a subject is provided with a Left Ventricular Assist Device (LVAD). LVAD comprises a pump 310 and connection conduits 311 and 312. The pump 310 pumps blood to the body and is a battery-operated, mechanical device, which surgically implanted inside a subject's chest. The LVAD helps maintain the pumping ability of a heart that is too weak to work on its own. The device supports the main pumping chamber, i.e., left ventricle, by moving blood to aorta and the rest of the body. The pump 310 is connected to the left ventricle through conduit 311 and to the aorta through conduit 312. The sensors 100 are arranged on the conduits 311 and 312 at the inlet and outlet of the pump 310. The conduits comprise small windows corresponding to the shape of the flexible membrane 120 (Fig. 2) in the base of the pressure sensor 100. The sensor's base is attached to the conduit and covers the window exposing the flexible membrane to the blood pressure in the conduits. The pressure sensors 100 are connected to a controller unit 1000 inside or outside the body of the subject by means of connection wires 140.

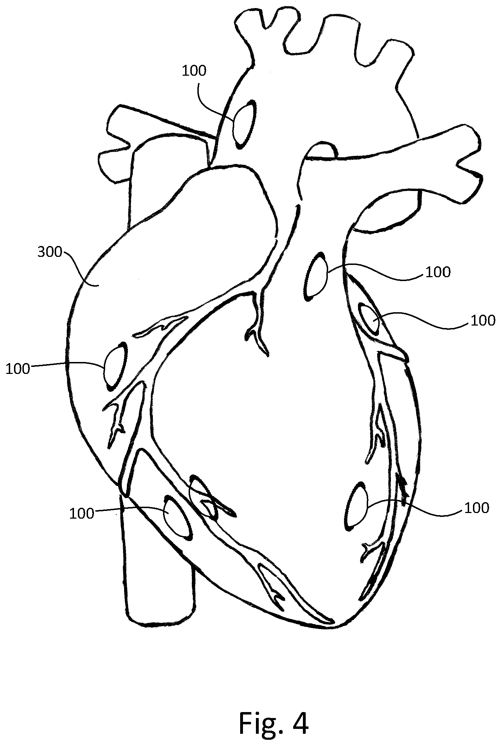

Fig. 4 illustrates a second exemplary application of the pressure sensor described previously. A heart 300 of a subject is provided with pressure sensors 100. In this example, the pressure sensors 100 are attached to (from the top of the figure) the aorta, pulmonary artery, right atrium, left atrium, right ventricle and left ventricle. These positions are given as examples and the pressure sensor can be positioned in any position from where a pressure value is required. The pressure sensors may be attached to the heart tissue myocardium by means of Dacron, ePTFE, polyester or attached in the epicardium. The pressure sensors in this example can be connected to a heart prosthesis such as a TAH or VAD system to regulate the flow in the device according to the value of blood pressure in the chamber/chambers of the heart 300.

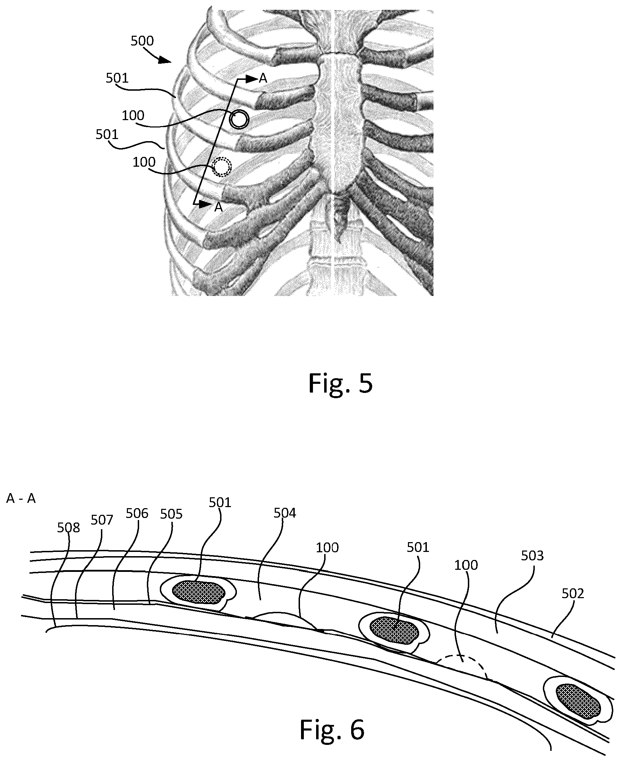

Figs. 5 and 6 illustrate a third application. Fig. 5 shows a portion of thorax 500 and Fig. 6 is a cross-sectional and very schematic view along line A-A in Fig. 5 but with the tissues included.

Designation sign 501 designates a rib, 502 skin, 503 superficial fascia, 504 intercostal muscles, 505 parietal pleura, 506 pleural cavity, 507 visceral pleura and 508 a lung.

In this application, the pressure sensors 100 are arranged between ribs 501 and in the intercostal muscles 504. The electrical connections, which may be extended out through the tissues are not illustrated. The pressure sensor may also be arranged in the vicinity of the costal cartilage.

In a TAH application or heart assist pump application, measuring the pressure of the thorax cavity may be significant. The pressure inside the thorax cavity continuously changes during the respiration. As the pressure inside the thorax cavity affects the pressure inside the atrium, it is desirable to measure the pressure inside the thorax cavity (reference pressure) as well. This way, it is possible to compensate for the altering of the pressure inside the thorax cavity. The pressure inside the thorax cavity can be measured by:
1. Using a pressure sensor assembled inside the pump but designed to measure the pressure inside the thorax cavity,
2. Using a separate pressure sensor placed inside the thorax cavity and connected to the pump by a wire, or
3. Arranging a pressure sensor outside the thorax cavity but inside the chest-wall and exactly in contact with parietal pleura layer 505 as in the above-mentioned example.

Thus, the pressure sensor 100 can be connected to the pleura (pleural) membrane, which will function as a natural flexible membrane between the thorax cavity and the pressure sensor. It may be enough to use one pressure sensor for measuring the pressure inside the thorax cavity for both the right and left pump to remove any effect of breathing and is measured outside of the heart itself. A control unit may receive input signals from the pressure sensors and output signals to the pump driver system. The signals may be received and provided directly by a wire.

The pressure sensor of the present invention may be implanted in or in communication with other body cavities or organs, such as kidneys, urinary bladder, abdominal cavity, etc. where a pressure value reading is desired.

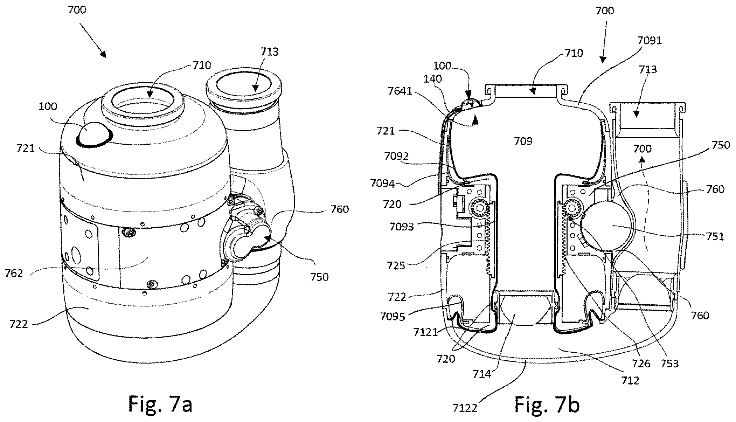

Figs. 7a and 7b illustrate a fourth exemplary application of the pressure sensor described previously. Fig. 7a illustrates a pump 700, one pump portion of a TAH, in perspective view and Fig. 7b is a cross-sectional view of the pump 700.

This embodiment comprises a first blood receiving part 700 of the artificial heart and the driver/actuation system 750 to driver/actuation the artificial heart to create the pumping mechanism. The artificial heart mainly consists of two pumps 700 the left pump and the right pump (not shown). Each pump comprises one blood receiving part and one driver/actuation system. The blood receiving part is assembled to the driver system in an easy way by screws, glue or in combination. The blood receiving part comprises an artificial atrium 709, and an artificial ventricle 712. Between the artificial atrium 709 and artificial ventricle 712 there is a connecting cylinder 720 with it is valve corresponding to the mitral valve in left side of the natural heart and to the tricuspid valve in the right side of the natural heart. The connecting cylinder can be made of flexible blood compatible material such as polyurethane, silicone or any other blood compatible material.

Each atrium 709 consists mainly of an atrial covering wall, which mostly consists of two layers an outer layer 7091, which is stiff and made of hard polyurethane, hard silicone, biocompatible metal such as titanium or stainless steel or any other biocompatible stiff material. Further, there is an inner flexible blood compatible membrane 7092 made of polyurethane, silicone or any other blood compatible material. The inner flexible membrane is an extension of a flexible atrial membrane, which is an extension of the flexible membrane 7093 lined the inner surface of the connecting AV-cylinder 720, which is an extension of a flexible ventricular membrane 7021. Further, there is an atrial assembling ring 721, which is stiff and made of stiff polyurethane, stiff silicone, biocompatible metal such as titanium or stainless steel or any other biocompatible firm material. The atrial assembling ring is assembled to the upper edge of the driver/actuation system by screws, glue or in combination. The atrium has an inlet opening 710 to let the blood flowing inside the atrium. There is a pressure window 764 with a diameter of, e.g., at least 5-30 mm with a circular, oval, or any other shape. The wall of the pressure window consists only of the flexible membrane without the stiff wall layer. This pressure window is a part of the pressure sensor 100 construction, as described earlier.

Each ventricle 712 consists mainly of a ventricular covering wall, which mostly consists of two layers an outer layer, which is stiff and made of stiff polyurethane, stiff silicone, biocompatible metal such as titanium or stainless steel or any other biocompatible stiff material. Further, there is an inner flexible blood compatible membrane 7121 made of polyurethane, silicone or any other blood compatible material. The inner flexible membrane is an extension of a flexible ventricular membrane, which is an extension of the flexible membrane 7093 lined the inner surface of the connecting AV-cylinder 720. Further, there is a ventricular assembling ring 722, which is a stiff and made of stiff polyurethane, stiff silicone, biocompatible metal such as titanium or stainless steel or any other biocompatible stiff material. The ventricular assembling ring assembled to the lower edge of the driver/actuator system by screws, glue or in combination.

The driver/actuator system 750 consists of a housing 762, a gearbox 753, and an electrical motor 751. The housing is made of a stiff plastic biocompatible material such as PEEK and any other biocompatible plastic material or biocompatible metal. The housing is enclosing the gearbox and electrical motor. The gearbox consists of multiple cogwheels and metal axles. The electrical motor is of type of brushless motor or any other type of electrical motor with or without an encoder. The pressure sensor is described in detail later in this application. The motor 751 is arranged between the outlet 713 and the pump, inside the pump housing. A spacer 760 may be arranged between the outlet 713 housing and the motor 751. The spacer 760 is made of a material with good thermal transfer capacity, such that when blood (or other liquid) flows 700 through the outlet pipe, the heat from the driving mechanism is transferred by means of the spacer 760 and the wall of the outlet 713 to the blood, which transfers and reduces the heat from the driving mechanism.

The center of the driver/actuation system has a cylindrical shape and enclosing AV-cylinder 720 which consists of a stiff plastic biocompatible material such as stiff polyurethane, stiff silicone, or a biocompatible metal such as titanium, stainless steel or any other biocompatible stiff material. The AV-cylinder is lined by a flexible membrane 7093 which is an extension of the flexible atrial membrane 7092 and flexible ventricular membrane 7021. The AV-cylinder encloses the valve 714. There are two racks, 725 and 726, one on each side of the AV-cylinder 720. Each rack is articulated with the cogwheel of the gearbox 753 to be actuated in upward and downward direction.

There may be a wire (not shown), which is connected to the driver/actuation system to supply the electrical motor with electrical power and control signal. Further, there may be a covering membrane (not shown), which is a layer of biocompatible plastic material such as polyurethane or silicone enclosing the whole driver/actuation system allowing the wire to the electrical motor and the pipe of the pressure sensor to penetrate through this covering membrane.

The sensor 100 construction comprises an opening 764 in the chamber's 709 wall, from which the flexible thin membrane 120 is exposed to pressure from the chamber 709. The pressure sensor pressure receiving portion is thus arranged in the left (or right) atrium 709 and right pump atria (or upper half of each pump) as described earlier. The flexible membrane 120 is arranged as a portion of the inner wall of the housing.

In operation, when the membrane 120 of the atrial wall 760 is affected by the pressure of the blood inside the atrium 709 of each pump, the flexible membrane 120 will either bulge to the inside of the pressure sensor 100 when the pressure inside the atrium increases or it will bow inwards to atrial cavity when the pressure decreases. In this way, the pressure transfer medium inside the housing and lumen of the pipe 163 compresses or will expand due to the influence of the pressure inside the atrium 709. The pressure is transferred inside the pipe 163 from the membrane to the sensor 734 surface. The sensitive surface of the MEMS sensor 150 is thus affected by the pressure changing in the pressure transfer medium and the MEMS sensor translates the pressure to a digital value and an electrical signal representing the pressure is generated and provided to the controller unit.

Many of the previously exemplified embodiments relate to a pulsating pump or a displacement pump; however, the method, sensor and control unit of the invention are likewise applicable in systems having different types of pump(s), e.g., a centrifugal pump or other continuous flow pump. The continuous flow pump may include one or several of canned, radial, side channel, regenerative turbine, axial, or diagonal pump type. A displacement pump may include one or several of dosing, progressing cavity, gear, multi screw, piston diaphragm, plunger and piston, rotary lobe, vacuum and hose pump type.

Consequently, the term "stroke volume" as used herein, relates to a specific amount of fluid displaced under a specific time interval and the term "heart rate" as used herein relates to a rate a pump displaces an amount of fluid.

The terms right or left atrium as used herein, relate to a compartment inside the pump housing into which a fluid is provided and discharged from.

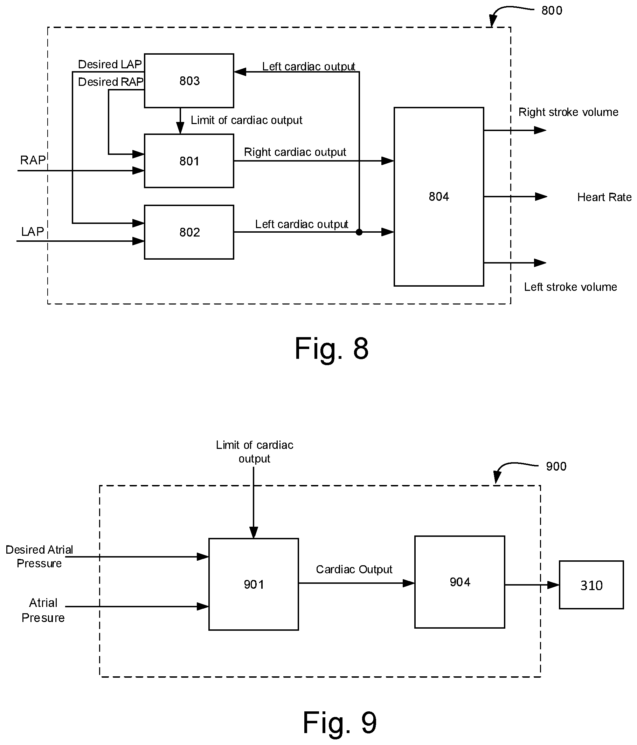

Fig. 8 illustrates the schematics and functional blocks of an exemplary control unit 800 for TAH applications. The control unit 800, according to this embodiment, may comprise functional blocks:
- a cardiac output control for right atrium 801,
- a cardiac output control for left atrium 802,
- a flow control block 803, and
- a processing block 804.

The main function of the control unit 800 is to set a heart rate and stroke volumes for either half (i.e., left and right pumps) of the blood-pump 700 to values that are appropriate to the current physiological state of the subject.

The control problem may be solved by dividing it into sub-problems, comprising:
- Flow control: This determines the correct flow limit and desired atrium pressures, which will be inputs to keep the cardiac output within appropriate ranges. Desired atrial pressures can be overridden, by allowing the operator of the prosthesis to set desired atrium pressures manually.
- Determining cardiac output of either pump to control atrium pressures: This is done individually with no inter-dependency between the pumps.
- Determining a heart rate: The heart rate and the cardiac output of either pump (calculated in the previous step) will in turn provide the stroke volumes for either pump.

The inputs to the functional block include:
- Right Atrium Pressure (RAP), measured by the pressure sensor 100 in the right atrium to (right) cardiac output control 801; and
- Left Atrium Pressure (LAP) measured by the pressure sensor 100 in the left atrium to (left) cardiac output control 802.

Determining a cardiac output of either pump may be carried out with only the atrium pressures as input.

The stroke volumes, as mentioned, may be calculated as stroke length multiplied with a constant, and may hence not agree with the actual pumped stroke volumes. This implies that the flows reported also are only approximations.

The functional block flow control 803 determines the flow limit and the desired atrium pressures. The flow limit is a boundary for the cardiac output of the right pump.

The objective of the flow control is to keep the flow of the right-side pump 802 low enough, so that the left pump 803 never reaches its maximum flow. If this happens, i.e., a maximum flow is reached, the left pump cannot keep LAP bounded, which would pose a risk of pulmonary oedema. Limiting the right pump's capacity may cause RAP to rise; this is however can be seen as acceptable. Higher RAP will also increase Central Venous Pressure (CVP), which will help reduce venous return. The flow control block 803 receives output from left cardiac output control 802 and sets the desired RAP, desired LAP, and limit of right-side cardiac output.

The actual flow of either half of the pump 700, may be dependent on various factors such as outflow and inflow pressures, even given the same heart rate and stroke length. Because of this, the flow limit of the right pump (700) cannot be set constant but has to be changed dynamically.

The controller unit 800 may be an implanted or integrated microcomputer or electronic chips. The microcomputer can provide the control signals to the pump actuating means to change its pumping activity. If for some reason the microcomputer is not receiving any input information, the pump actuating means may continue at a constant level of activity. Fig. 9 is a schematic illustration of blocks of an alternative exemplary controller unit 900, e.g., for heart assist pump (VAD, LVAD, etc.) applications. The controller unit 900, according to this embodiment, may comprise functional blocks:
- a cardiac output control 901, and
- a processing block 904.

The main function of the control unit 900 may be to set the speed of the blood-pump 310 to values that are appropriate to the current physiological state of the subject.

While the controller unit 800 of the embodiment according to Fig. 8 may be suitable for pulsatile pumps, controller unit 900 of Fig. 9 may be suitable for continuous flow pumps. However, the controller unit 800 may also be configured to set pulsation speed and thus the pump speed.

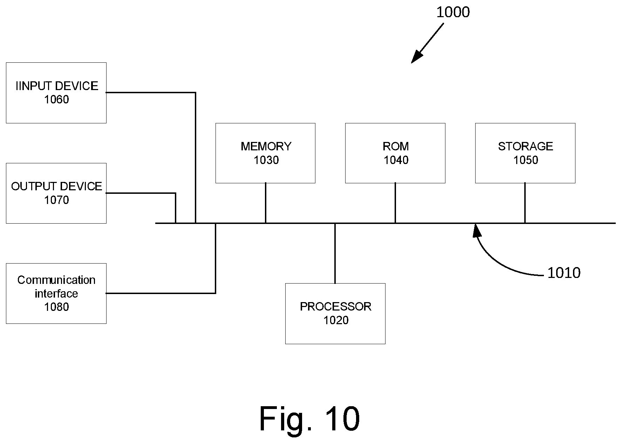

Fig. 10 is a diagram of an exemplary controller unit 1000 in which methods described herein may be implemented. The controller unit 1000 may include a bus 1010, a processor 1020, a memory 1030, a read-only memory (ROM) 1040, a storage device 1050, an input device 1060, an output device 1070, and a communication interface 1080. Bus 1010 permits communication among the components of the controller unit 1000. The controller unit 1000 may also include one or more power supplies (not shown). One skilled in the art would recognize that the controller unit 1000 may be configured in a number of other ways and may include other or different elements.

The processor 1020 may include any type of processor or microprocessor that interprets and executes instructions. Processor 1020 may also include logic that is able to decode media files and generate output to, for example, to a speaker, a display, etc. Memory 1030 may include a random-access memory (RAM) or another dynamic storage device that stores information and instructions for execution by processor 1020. Memory 1030 may also be used to store temporary variables or other intermediate information during the execution of instructions by processor 1020.

ROM 1040 may include a conventional ROM device and/or another static storage device that stores static information and instructions for processor 1020. Storage device 1050 may include a magnetic disk, solid state drive or optical disk and its corresponding drive and/or some other type of recording medium and its corresponding drive for storing information and instructions. Storage device 1050 may also include a flash memory (e.g., an electrically erasable programmable read only memory (EEPROM)) device for storing information and instructions.

Input device 1060 may include one or more conventional mechanisms that permit a user to input information to the controller unit 1000, such as a keyboard, a keypad, a directional pad, a mouse, a pen, voice recognition, a touchscreen and/or biometric mechanisms, etc. Output device 1070 may include one or more conventional mechanisms that output information to the user, including a display, a printer, one or more speakers, etc. Communication interface 1080 may include any transceiver-like mechanism that enables controller unit 1000 to communicate with other devices and/or systems. For example, communication interface 1080 may include a modem or an Ethernet interface to a LAN. Alternatively, or additionally, communication interface 1080 may include other mechanisms for communicating via a network, such as a wireless network. For example, a communication interface may include a radio frequency (RF) transmitter and receiver and one or more antennas for transmitting and receiving RF data.

The controller unit 1000, consistent with the invention, provides a platform through which a pump is controlled as described earlier. According to an exemplary implementation, the controller unit 1000 may perform various processes in response to processor 1020 executing sequences of instructions contained in memory 1030. Such instructions may be read into memory 1030 from another computer-readable medium, such as storage device 1050, or from a separate device via communication interface 1080. It should be understood that a computer-readable medium may include one or more memory devices or carrier waves. Execution of the sequences of instructions contained in memory 1030 causes processor 1020 to perform the acts that have been described earlier. In alternative embodiments, hard-wired circuitry may be used in place of or in combination with software instructions to implement aspects consistent with the invention. Thus, the invention is not limited to any specific combination of hardware circuitry and software.

It should be noted that the word "comprising" does not exclude the presence of other elements or steps than those listed and the words "a" or "an" preceding an element do not exclude the presence of a plurality of such elements. It should further be noted that any reference signs do not limit the scope of the claims, that the invention may be implemented at least in part by means of both hardware and software, and that several "means", "units" or "devices" may be represented by the same item of hardware.

The abovementioned and described embodiments are only given as examples and should not be limiting to the present invention. Other solutions, uses, objectives, and functions within the scope of the invention as claimed in the below described patent claims should be apparent to the person skilled in the art.

The various embodiments of the present invention described herein is described in the general context of method steps or processes, which may be implemented in one embodiment by a computer program product, embodied in a computer-readable medium, including computer-executable instructions, such as program code, executed by computers in networked environments. A computer-readable medium may include removable and non-removable storage devices including, but not limited to, Read Only Memory (ROM), Random Access Memory (RAM), compact discs (CDs), digital versatile discs (DVD), Solid State Drive, etc. Generally, program modules may include routines, programs, objects, components, data structures, etc. that perform particular tasks or implement particular abstract data types. Computer-executable instructions, associated data structures, and program modules represent examples of program code for executing steps of the methods disclosed herein. The particular sequence of such executable instructions or associated data structures represents examples of corresponding acts for implementing the functions described in such steps or processes.

Software and web implementations of various embodiments of the present invention can be accomplished with standard programming techniques with rule-based logic and other logic to accomplish various database searching steps or processes, correlation steps or processes, comparison steps or processes and decision steps or processes. It should be noted that the words "component" and "module," if used herein and in the following claims, is intended to encompass implementations using one or more lines of software code, and/or hardware implementations, and/or equipment for receiving manual inputs.

## Claims

1. A pressure sensor (100) comprising:
- a housing (110) having one open portion,
- a flexible membrane (120) covering the open portion of the housing (110),
- a space (170) formed by said housing and the flexible membrane,
- the space (170) entirely filed with a pressure-transferring medium,
- a pressure sensitive sensor (150) attached to an inner surface of the housing via a circuit board (160) and having one surface to receive the pressure transferred from the membrane (120) the pressure transferring medium,
- the housing is made of biocompatible material and the flexible membrane is made of a biocompatible flexible material;
**characterised in that**
- the housing further comprises an attachment ring (130) at least partly surrounding the open portion of the housing and the flexible membrane, and configured to attach the pressure sensor to a surface of an object, a pressure value associated with which is to be measured,
- the attachment ring (130) is made of biocompatible material; and
- an electrical connection wire (140) configured to connect the pressure sensitive sensor to a control unit (1000).

2. The sensor of claim 1, wherein the sensor housing (110) is made of one of hard polyurethane, hard silicone, biocompatible metal comprising one of titanium or stainless steel or any other biocompatible stiff material.

3. The sensor of claim 1 or 2, wherein the attachment ring (130) is made of a biocompatible material, comprising one of Dacron, ePTFE, polyester or other material that can be healed together with natural tissues.

4. The sensor according to any of preceding claims, wherein the pressure sensitive sensor (150) comprises MEMS (small-scale microelectromechanical system) sensor or nanoelectromechanical systems (NEMS).

5. The sensor according to any of preceding claims, wherein the flexible membrane (120) is made of polyurethane, silicone, or any other blood compatible material.

6. The sensor according to any of preceding claims, wherein the pressure transferring medium in the housing consists of a biocompatible, implantable oil, or medical graded silicone oil, or any other biocompatible, medical graded fluid.

7. The sensor according to any of preceding claims, for use in or in communication with one or several of heart prothesis, total artificial heart, heart assist pumps, as an implant for measuring venous, atrial or arterial pressure in pulmonary and systemic circulations, thorax pressure measurement, kidneys, urinary bladder, or intestinal.

8. A controller unit (800, 900, 1000) for controlling a cardiac pump (700, 310), the cardiac pump comprising:
• at least one pump portion (700, 310),
• an inlet (710) connected to said at least one pump portion,
• an outlet (713) connected to said at least one pump portion,
• a pressure sensor (100) according to any of claims 1-7 configured to measure pressure of a fluid flowing from the inlet to the outlet,
• a pump actuator (750) configured to induce the flow of the fluid flow, and
the controller unit further comprises a memory (1030) and a processing unit (1020),
**characterized in that** the processing unit is configured to:
- receive a pressure value from the pressure sensor,
- receive a desired value for the pressure of the fluid flowing into the pump,
- calculate an error signal equal to the difference of desired value for the pressure and the measured pressure, and
- control the output of the pump such that the measured pressure is near or equal to the desired pressure, by controlling a pump control parameter, the pump control parameter comprising one or several of a pump stroke rate and/or a pump stroke volume and/or pump speed.

9. A cardiac prosthesis (700) comprising a pressure sensor according to any of claims 1-7.

## Patentansprüche

1. Drucksensor (100), umfassend:
- ein Gehäuse (110) mit einem offenen Abschnitt,
- eine flexible Membran (120), die den offenen Abschnitt des Gehäuses (110) bedeckt,
- einen Raum (170), der durch das Gehäuse und die flexible Membran gebildet ist,
- den Raum (170), der vollständig mit einem Druckübertragungsmedium gefüllt ist,
- einen druckempfindlichen Sensor (150), der über eine Leiterplatte (160) an einer inneren Oberfläche des Gehäuses befestigt ist und eine Oberfläche zum Aufnehmen des von der Membran (120) übertragenen Drucks des Druckübertragungsmediums aufweist,
- das Gehäuse besteht aus biokompatiblem Material und die flexible Membran besteht aus einem biokompatiblen flexiblen Material;
**dadurch gekennzeichnet, dass**
- das Gehäuse ferner einen Befestigungsring (130) umfasst, der den offenen Abschnitt des Gehäuses und die flexible Membran zumindest teilweise umgibt und konfiguriert ist, um den Drucksensor an einer Oberfläche eines Objekts zu befestigen, dessen zugehöriger Druckwert gemessen werden soll,
- der Befestigungsring (130) aus biokompatiblem Material besteht; und
- ein elektrischer Anschlussdraht (140) konfiguriert ist, um den druckempfindlichen Sensor mit einer Steuereinheit (1000) zu verbinden.

2. Sensor nach Anspruch 1, wobei das Sensorgehäuse (110) aus einem von hartem Polyurethan, hartem Silikon, biokompatiblem Metall, das entweder Titan oder Edelstahl umfasst, oder einem anderen biokompatiblen steifen Material besteht.

3. Sensor nach Anspruch 1 oder 2, wobei der Befestigungsring (130) aus einem biokompatiblen Material besteht, umfassend eines von Dacron, ePTFE, Polyester oder ein anderes Material, das mit natürlichem Gewebe zusammenheilen kann.

4. Sensor nach einem der vorstehenden Ansprüche, wobei der druckempfindliche Sensor (150) einen MEMS-Sensor (MEMS - small-scale microelectromechanical system) oder nanoelektromechanische Systeme (NEMS) umfasst.

5. Sensor nach einem der vorstehenden Ansprüche, wobei die flexible Membran (120) aus Polyurethan, Silikon oder einem anderen blutverträglichen Material besteht.

6. Sensor nach einem der vorstehenden Ansprüche, wobei das Druckübertragungsmedium in dem Gehäuse aus einem biokompatiblen implantierbaren Öl oder Silikonöl medizinischer Qualität oder einem anderen biokompatiblen Fluid medizinischer Qualität besteht.

7. Sensor nach einem der vorstehenden Ansprüche zur Verwendung in oder in Verbindung mit einem oder mehreren von Herzprothesen, Kunstherzen, Herzunterstützungspumpen, als Implantat zur Messung des venösen, atrialen oder arteriellen Drucks im Lungen- und Körperkreislauf, zur Messung des Thoraxdrucks, Nieren, Harnblase oder Darm.

8. Steuereinheit (800, 900, 1000) zum Steuern einer Herzpumpe (700, 310), wobei die Herzpumpe Folgendes umfasst:
• mindestens einen Pumpenabschnitt (700, 310),
• einen Einlass (710), der mit dem mindestens einen Pumpenabschnitt verbunden ist,
• einen Auslass (713), der mit dem mindestens einen Pumpenabschnitt verbunden ist,
• einen Drucksensor (100) nach einem der Ansprüche 1 bis 7, der konfiguriert ist, um den Druck eines Fluids zu messen, das vom Einlass zum Auslass strömt,
• einen Pumpenaktuator (750), der konfiguriert ist, um die Strömung des Fluidstroms zu induzieren, und
die Steuereinheit umfasst ferner einen Speicher (1030) und eine Verarbeitungseinheit (1020), **dadurch gekennzeichnet, dass** die Verarbeitungseinheit konfiguriert ist zum:
- Empfangen eines Druckwerts von dem Drucksensor,
- Empfangen eines Sollwerts für den Druck des in die Pumpe strömenden Fluids,
- Berechnen eines Fehlersignals, das der Differenz zwischen dem Sollwert für den Druck und dem gemessenen Druck entspricht, und
- Steuern der Leistung der Pumpe derartig, dass der gemessene Druck nahe am oder gleich dem Sollwert für den Druck liegt, indem ein Pumpensteuerungsparameter gesteuert wird, wobei der Pumpensteuerungsparameter einen oder mehrere der folgenden Parameter umfasst: Pumpenhubrate und/oder Pumpenhubvolumen und/oder Pumpendrehzahl.

9. Herzprothese (700), umfassend einen Drucksensor nach einem der Ansprüche 1 bis 7.

## Revendications

1. Capteur de pression (100) comprenant :
- un boîtier (110) comportant une partie ouverte,
- une membrane souple (120) recouvrant la partie ouverte du boîtier (110),
- un espace (170) formé par ledit boîtier et la membrane souple,
- l'espace (170) entièrement rempli d'un fluide de transfert de pression,
- un capteur sensible à la pression (150) fixé à une surface intérieure du boîtier par l'intermédiaire d'une carte de circuit imprimé (160) et ayant une surface pour recevoir la pression transférée par la membrane (120) du milieu de transfert de la pression,
- le boîtier est constitué d'un matériau biocompatible et la membrane souple est constituée d'un matériau souple biocompatible ;
**caractérisé en ce que**
- le boîtier comprend en outre un anneau de fixation (130) entourant au moins partiellement la partie ouverte du boîtier et la membrane souple, et conçu pour fixer le capteur de pression à une surface d'un objet dont la valeur de pression associée doit être mesurée,
- l'anneau de fixation (130) est constitué d'un matériau biocompatible ; et
- un fil de connexion électrique (140) conçu pour connecter le capteur sensible à la pression à une unité de commande (1000).

2. Capteur selon la revendication 1, dans lequel le boîtier de capteur (110) est constitué de l'un parmi le polyuréthane dur, silicone dur, métal biocompatible comprenant l'un parmi le titane ou l'acier inoxydable ou tout autre matériau rigide biocompatible.

3. Capteur selon la revendication 1 ou 2, dans lequel l'anneau de fixation (130) est constitué d'un matériau biocompatible, comprenant l'un parmi le Dacron, ePTFE, polyester ou autre matériau qui peut être cicatrisé avec les tissus naturels.

4. Capteur selon l'une quelconque des revendications précédentes, dans lequel le capteur sensible à la pression (150) comprend un capteur MEMS (système microélectromécanique à petite échelle) ou un système nanoélectromécanique (NEMS).

5. Capteur selon l'une quelconque des revendications précédentes, dans lequel la membrane souple (120) est constituée de polyuréthane, silicone, ou tout autre matériau compatible avec le sang.

6. Capteur selon l'une quelconque des revendications précédentes, dans lequel le milieu de transfert de pression dans le boîtier est constitué d'une huile implantable biocompatible, d'une huile de silicone de qualité médicale ou de tout autre fluide biocompatible de qualité médicale.

7. Capteur selon l'une quelconque des revendications précédentes, pour une utilisation dans ou en communication avec une ou plusieurs prothèses cardiaques, un cœur artificiel total, des pompes d'assistance cardiaque, en tant qu'implant pour la mesure de la pression veineuse, auriculaire ou artérielle dans les circulations pulmonaire et systémique, la mesure de la pression thoracique, les reins, la vessie urinaire ou l'intestin.

8. Unité de commande (800, 900, 1000) destinée à commander une pompe cardiaque (700, 310), la pompe cardiaque comprenant :
• au moins une partie de pompe (700, 310),
• une entrée (710) reliée à ladite au moins une partie de pompe,
• une sortie (713) reliée à ladite au moins une partie de pompe,
• un capteur de pression (100) selon l'une quelconque des revendications 1 à 7, configuré pour mesurer la pression d'un fluide s'écoulant de l'entrée à la sortie,
• un actionneur de pompe (750) configuré pour induire l'écoulement du flux de fluide, et
l'unité de commande comprend en outre une mémoire (1030) et une unité de traitement (1020), **caractérisée en ce que** l'unité de traitement est configurée pour :
- recevoir une valeur de pression du capteur de pression,
- recevoir une valeur souhaitée pour la pression du fluide entrant dans la pompe,
- calculer un signal d'erreur égal à la différence entre la valeur souhaitée pour la pression et la pression mesurée, et
- commander la sortie de la pompe de telle sorte que la pression mesurée soit proche ou égale à la pression souhaitée, en commandant un paramètre de commande de la pompe, le paramètre de commande de la pompe comprenant un ou plusieurs parmi un taux de course de pompe et/ou volume de course de pompe et/ou vitesse de pompe.

9. Prothèse cardiaque (700) comprenant un capteur de pression selon l'une quelconque des revendications 1 à 7.
